(19) Europäisches Patentamt
European Patent Office
Office européen des brevets

(11) **EP 0 736 514 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**13.10.2010 Patentblatt 2010/41**

(51) Int Cl.:
***C07C 201/16*** *(2006.01)*

(45) Hinweis auf die Patenterteilung:
**20.06.2001 Patentblatt 2001/25**

(21) Anmeldenummer: **96104233.0**

(22) Anmeldetag: **16.03.1996**

(54) **Rückgewinnung von Salpetersäure aus Nitrierprozessen**

Nitric acid recovery from nitriding processes

Récupération d'acide nitrique dans les procédés de nitruration

(84) Benannte Vertragsstaaten:
**AT BE CH DE ES FR GB IT LI NL PT SE**

(30) Priorität: **04.04.1995 DE 19512114**

(43) Veröffentlichungstag der Anmeldung:
**09.10.1996 Patentblatt 1996/41**

(73) Patentinhaber: **Josef Meissner GmbH & Co.**
**D-50968 Köln (DE)**

(72) Erfinder:
 • **Hermann, Heinrich, Dr.**
   **D-50968 Köln (DE)**
 • **Gebauer, Jürgen**
   **D-53840 Troisdorf (DE)**

(74) Vertreter: **Gesthuysen, von Rohr & Eggert**
**Patentanwälte**
**Postfach 10 13 54**
**45013 Essen (DE)**

(56) Entgegenhaltungen:
**EP-A- 0 155 586     EP-A- 0 279 312**
**EP-A- 0 415 354     WO-A-92/11227**
**CA-A- 1 034 603     GB-A- 125 140**
**US-A- 3 221 064     US-A- 4 257 986**
**US-A- 5 057 632**

 • **DATABASE WPI Week 8151 Derwent Publications Ltd., London, GB; AN 81-93774d XP002002902 & JP-A-56 142 245 (MITSUBISHI CHEM IND KK) , 6.November 1981**

EP 0 736 514 B2

**Beschreibung**

Die vorliegende Erfindung betrifft ein Verfahren zum Entfernen und Wiedergewinnen von Salpetersäure, Schwefelsäure und Stickoxiden aus den bei der Nitrierung von Toluol oder Mononitrotoluolen (MNT) nach Abtrennung der Nitriersäure anfallenden rohen Dinitrotoluole (DNT).

DNT wird aus Toluol in einem zweistufigen Verfahren im Gegenstrom hergestellt, wobei in den Nitriersäuren stets ein Überschuß an Salpetersäure von 1,01 bis 1,08 der benötigten Menge vorhanden sein muß, um einen quantitativen Umsatz der zu nitrierenden Stoffe (Toluol bzw. MNT.) auf der jeweiligen Stufe zu erreichen. In der ersten Nitrierstufe (MNT-Stufe) wird bei Temperaturen zwischen 35 und 45 °C Toluol mit der DNT-Endsäure aus der DNT-Stufe und frischer Salpetersäure selektiv zu MNT umgesetzt.

Die nach beendeter Reaktion in der ersten Nitrierstufe anfallende Nitriersäure (MNT-Endsäure) enthält im Gleichgewicht mit der organischen Phase noch 70 bis 72 % Schwefelsäure, ca. 0,3 bis 0,7 % Salpetersäure und 0,4 bis 2,0 % Nitrose als $HNO_2$. Nach Phasentrennung wird die MNT-Endsäure einer MNT-Endsäurebehandlung zugeführt, um noch die Restmenge an Salpetersäure, die Nitrose und das gelöste MNT/DNT zu entfernen.

Das Roh-MNT, das noch Nitrokresole, Salpetersäure, Nitrose und andere Abbauprodukte enthält, wird direkt ohne Reinigung in der zweiten Stufe mit einer frischen Mischsäure aus 96 % Schwefelsäure und etwa 98 % bzw. 63 bis 67 % Salpetersäure zum DNT umgesetzt.

Das nach Abtrennung der Nitriersäure (DNT-Endsäure) anfallende Roh-DNT enthält neben dem DNT-Isomerengemisch, aus etwa 76 % 2,4-DNT, 19,5 % 2,6-DNT, 0,6 % 2,5-DNT, 3,7 % "Ortho-Isomere" (2,3-und 3,4-DNT) und 0,08 % 3,5-DNT, max. 0,01 % MNT und Spuren von TNT (z.B. 0,02 %) noch Nitrokresole, Salpetersäure, Schwefelsäure und gelöstes Stickstoffdioxid ($NO_2$) (s. US-Patent 4,482,769). Die Menge der im Roh-DNT gelösten und suspendierten Salpetersäure, Schwefelsäure und Nitrose hängt von der Zusammensetzung der DNT-Endsäure und der Qualität der Phasentrennung zwischen der DNT-Endsäure und dem Roh-DNT nach beendeter Umsetzung ab. Um diese Stoffe zu entfernen, wird das Roh-DNT einer Wäsche zugeführt.

Die Wäsche wird bei 60 bis 70 °C in drei Stufen durchgeführt, wobei in der ersten Stufe (saure Wäsche) mit Wasser alle Säuren wie (Schwefelsäure, Salpetersäure, Nitrose) ausgewaschen werden. In der zweiten Waschstufe (Alkaliwäsche) werden mit Natriumcarbonat-Lösungen alle schwach sauren Stoffe wie z.B. die Nitrokresole ausgewaschen. In der letzten Waschstufe werden mit Wasser die Spuren an Natriumcarbonat und Kresolen entfernt.

Die mit DNT gesättigten vereinigten Abwässer aus der Wäsche des Roh-DNT sind aufgrund des hohen Gehaltes an Säuren im Roh-DNT stets sauer und enthalten je nach der Menge des Waschwassers (1,5 - 3 $m^3$/DNT) im Verhältnis zu dem gewaschenen DNT etwa 0,2 bis 3,0 % Schwefelsäure, 1 bis 3 % Salpetersäure, 0,1 bis 0,2 % $HNO_2$, 0,2 bis 0,5 % DNT (je nach Temperatur und Säuregehalt), 400 bis 800 ppm Nitrokresole und andere Abbauprodukte aus der Oxidation der Nitrokresole in der DNT-Stufe.

Neben den toxischen Nitrokresolen, die vor Abgabe des Abwassers in einen Vorfluter entfernt werden müssen, stellt auch der hohe Gehalt an Salpetersäure von 1 bis 3 % und Schwefelsäure von 0,2 bis 3 % im Abwasser ein Problem dar.

Sowohl die im Abwasser vorhandene Salpetersäuremenge als auch die in der Abfallsäure vorhandene Restsalpetersäuremenge und Nitrose sind für den Nitrierprozeß verloren und fallen in Abhängigkeit von der Behandlungsmethodik als mehr oder weniger verdünnte Salpetersäure an, die nicht unmittelbar in die Nitrierung zurückgeführt werden kann. So wird aus einer MNT-Endsäure durch flush-Verdampfung im Vakuum eine dünne Salpetersäure mit ca. 16 % Salpetersäure erhalten oder durch Strippen bei Normaldruck ein 2-Phasen-Gemisch mit 15 bis 30 % Salpetersäure und MNT/DNT als organischer Phase erhalten.

Vor Abgabe dieser Abwässer aus der sauren Wäsche des DNT bzw. aus der Reinigung der MNT-Endsäure in einen Vorfluter müssen nicht nur das gelöste MNT/DNT sondern auch der hohe Nitrat- und Sulfatgehalt auf die gesetzlich gültigen Grenzwerte für die Einleitung von Abwässern (z.B. 50 mg/$m^3$ für Nitrat, 1200 mg für Sulfat und CSB 175 mg $O_2$/l), reduziert werden. Dies ist nur möglich durch eine Behandlung des Abwassers mit Calciumhydroxid zum Zwecke der Abtrennung des überschüssigen Sulfats als Calciumsulfat, mit nachfolgendem biologischem Abbau des Nitrates und der organischen Belastung.

Um diesen Verlust an Salpetersäure von ca. 8 % der für die Nitrierung benötigten Menge und damit den Aufwand bei der Entfernung der Salpetersäure aus dem Abwasser zu reduzieren, wurde vorgeschlagen, die z.B. bei der Reinigung der MNT-Endsäure durch eine flush-Verdampfung anfallende dünne Salpetersäure von ca. 16,3 % Salpetersäure und ca. 4,9 % $HNO_2$ durch Destillation auf eine 65 %ige Salpetersäure aufzukonzentrieren, die wieder in die Nitrierung zurückgeführt werden kann (EP-B-415 354).

Weiterhin wurde versucht, durch eine Wäsche des Roh-DNT mit bis zu 10 % Wasser die im Roh-DNT gelöste bzw. suspendierte Salpetersäure, Schwefelsäure und Nitrose so zurückzugewinnen, daß die bei dieser Wäsche anfallende konzentrierte Waschsäure mit einer Dichte über der des DNT unmittelbar in die Nitrierung zurückgeführt werden kann (EP-B-279 312).

Durch diese Verfahrensweise können etwa 50 bis 72 % der im Roh-DNT gelösten Salpetersäure zurückgewonnen werden. Aber die noch im DNT verbleibende Menge an Salpetersäure von 0,3 bis 0,6 % (3 - 6 kg/t DNT) gelangen weiterhin über die Wäsche ins Abwasser und stellen eine erhebliche Belastung dar.

Zur Rückgewinnung der Salpetersäure aus dem Roh-DNT wurde ferner versucht (US-PS-4 257 986), durch Wäsche des Roh-DNT mit einer gereinigten MNT-Endsäure einen Teil der Salpetersäure aus dem Roh-DNT zu extrahieren und anschließend diese mit Salpetersäure beladene MNT-Endsäure wieder in die Nitrierung zurückzuführen. Mit diesem Verfahren können die im Roh-DNT gelöste Salpetersäure und Nitrose nicht vollständig, sondern nur entsprechend dem Verteilungsgleichgewicht für diese Stoffe zwischen dem DNT und der Säure extrahiert werden (ca. 50 - 60 % der vorhandenen Salpetersäure).

Weiterhin wurde versucht, durch Änderung der Nitrierbedingungen die Verluste an Salpetersäure durch Extraktion mit dem Roh-DNT bzw. die Bildung von Nitrose aus Salpetersäure durch Oxidation der im Roh-MNT enthaltenen Dinitrokresole (vorwiegend 2,4-Dinitroparakresol) zu reduzieren, indem die Nitrierung des Toluols zum MNT nicht bei ca. 70 bis 72 % sondern bei 72 bis 76 % Schwefelsäure in der MNT-Endsäure durchgeführt wird (EP-B-155 586).

Durch diese Nitrierung des Toluols zum MNT in einer MNT-Endsäure mit einem Schwefelsäuregehalt von ca. 75 % werden bis zu 25 % weniger Dinitrokresol gebildet als bei der Nitrierung in einer MNT-Endsäure von 70 % Schwefelsäure (Hanson et al, ACS Symposium Series No. 22, 132 (1976) Industrial and Laboratory Nitration).

Alle diese Maßnahmen tragen dazu bei, die Verluste an Salpetersäure bei der Nitrierung von Toluol zu DNT durch Austrag aus dem Nitrierprozeß zu reduzieren. Aber sowohl die Wäsche des Roh-DNT nach der EP-B-279 312 als auch die Aufkonzentrierung der aus der flush-Verdampfung von MNT-Endsäure erhaltenen Salpetersäure nach der EP-B-415 354 führt auf den jeweiligen Teilstufen zu einer Reduzierung des Salpetersäureverlustes. Bezogen auf den Gesamtprozeß (Nitrierung, Endsäurebehandlung und Wäsche) werden immer noch Abwässer mit hohen Nitratkonzentrationen erhalten.

Bei diesen bekannten Maßnahmen handelt es sich um Teillösungen, um den Verlust an Salpetersäure für die Nitrierung und damit die Belastung des Abwassers aus einer DNT-Anlage mit Nitrat zu vermindern.

Die canadische Patentschrift CA-A-1 034 603 beschreibt ein Verfahren zur Herstellung von Toluolidendiamin, bei dem Toluol in Gegenwart einer anorganischen Säure nitriert wird und danach die so gebildeten Rohdinitrotoluole mit Wasser, das frei von säureneutralisierenden Verbindungen sein soll, gewaschen werden, um die überschüssigen Säuren vor der katalytischen Reduzierung der Dinitrotoluole zu den Diaminen zu entfernen. Der gesamte resultierende wäßrige Extrakt fällt anschließend als Abwasser an. Eine Wiederverwendung und Rückführung des Extraktes kommt bei dem Verfahren nach der CA-A-1 034 603 aus Rentabilitätsgründen nicht in Betracht, weil das gesamte Waschwasser mit so geringer Konzentration an Schwefelsäure, Salpetersäure, salpetriger Säure und Nitroaromaten anfällt, daß ein Rezyklieren gänzlich ausscheidet. Die Waschlösung ist also derart verdünnt, daß ein Aufkonzentrieren nicht lohnt. Deshalb wird sie als Abwasser verworfen.

Der Erfindung liegt die Aufgabe zugrunde, die Wäsche des Roh-DNT zur Entfernung der darin gelösten Salpetersäure, Schwefelsäure und Stickoxide so durchzuführen, daß diese Säuren nahezu vollständig wiedergewonnen werden und das Abwasser nicht mehr belasten.

Gegenstand der Erfindung ist ein Verfahren zum Entfernen und Wiedergewinnen von Salpetersäure, Schwefelsäure und Stickoxiden aus den bei der Nitrierung von Toluol oder Mononitrotoluolen nach Abtrennung der Nitriersäure anfallenden rohen Dinitrotoluolen nach Patentanspruch 1; weitere Ausgestaltungen des erfindungsgemäßen Verfahrens sind Gegenstand der Verfahrensunteransprüche (Patentansprüche 2 bis 7). Erfindungsgemäß wird bei der Entfernung und Wiedergewinnung von Salpetersäure, Schwefelsäure und Stickoxiden aus den bei der Nitrierung von Toluol oder Mononitrotoluolen nach Abtrennung der Nitriersäure anfallenden rohen Dinitrotoluolen derart vorgegangen, daß die rohen Dinitrotoluole mit einer verdünnten wäßrigen Lösung von Salpetersäure, Schwefelsäure und salpetriger Säure, deren Dichte geringer ist als die der Dinitrotoluole, mehrstufig, insbesondere zwei- bis vierstufig, im Gegenstrom extrahiert werden, wobei das Volumenverhältnis der Dinitrotoluole zu der wäßrigen Lösung jeweils 1 : 3 bis 10 : 1, vorzugsweise 1 : 1 bis 4 : 1, beträgt und der wäßrige Extrakt direkt oder nach Aufkonzentrierung in die Nitrierung zurückgeführt wird. In allen Extraktionsstufen wird zweckmäßig bei einer Temperatur über der Schmelztemperatur der Dinitrotoluole gearbeitet, und die Dichte der wäßrigen Lösung sollte in allen Stufen geringer sein als die der Dinitrotoluole. Die verdünnte wäßrige Lösung wird innerhalb jeder Extraktionsstufe im Kreislauf geführt. Das gewünschte Volumenverhältnis der Dinitrotoluole zu der verdünnten wäßrigen Lösung von Salpetersäure, Schwefelsäure und salpetriger Säure wird über die Zugabe von Frischwasser in den Extraktionskreislauf der verdünnten Lösung der letzten Extraktionsstufe eingestellt. Hierbei wird insbesondere das bei der Aufkonzentrierung des wäßrigen Extraktes anfallende Kondensat zugesetzt. Die aus der ersten Extraktionsstufe abgezogene wäßrige Lösung ist ein Salpetersäure/Schwefelsäuregemisch mit 25 bis 40 % Gesamtsäure. Diese wird allein oder vorzugsweise zusammen mit der bei der Mononitrotoluolendsäurebehandlung anfallenden Salpetersäure bis zu einem Gesamtsäuregehalt von 65 %, berechnet als $HNO_3$, aufkonzentriert.

Es ist überraschend, daß nach dem erfindungsgemäßen Verfahren die Salpetersäure, die Nitrose- und die Schwefelsäure in etwa 98%iger Ausbeute aus dem Roh-DNT extrahiert werden können, weil bei der Wäsche des Roh-DNT mit einer Säure mit einem Salpetersäure-/ Schwefelsäuregemisch mit 25 bis 40 % Gesamtsäure im Gleichgewicht mit dem zu waschenden DNT Salpetersäure bereits wieder in nicht zu vernachlässigenden Mengen in die organische Phase zurückextrahiert wird. So wird z. B. aus 25 Gew.-%iger Salpetersäure im Gleichgewicht mit DNT bei 60 °C bereits wieder soviel Salpetersäure in das flüssige DNT zurückextrahiert, daß ein DNT mit ca. 0,6 % an Salpetersäure erhalten wird.

Weiterhin ist überraschend, daß bei erfindungsgemäßem Vorgehen auch das im Roh-DNT gelöste $NO_x$ ($NO_2$) nach der Gleichung

$$6NO_2 \xrightarrow{+ 2H_2O} 4HNO_3 + 2NO$$

fast vollständig als Salpetersäure zurückgewonnen werden kann. Das $NO_2$ wird also nicht erst in der alkalischen zweiten Waschstufe einer Wäsche von Roh-DNT ausgewaschen und damit das Abwasser entlastet. Das Stickstoffmonoxid wird wie üblich aufgefangen und kann nach Aufoxidation auch wieder für die Nitrierung genutzt werden.

Die in der letzten Waschstufe der sauren Wäsche des Roh-DNT zugegebene Frischwassermenge hängt nicht nur von der gewählten Konzentration an Säure auf der ersten Waschstufe (z.B. 30 % Gesamtsäure als Salpetersäure), sondern auch von der Menge und dem Verhältnis der im Roh-DNT gelösten Salpetersäure/ Schwefelsäure/$NO_2$ ab.

Zur Aufrechterhaltung des Verhältnisses von DNT zur wäßrigen Lösung innerhalb der einzelnen Waschstufen wird nach Phasentrennung DNT/Waschsäure die abgetrennte Waschsäure in die Waschstufe zurückgeführt und nur der Überschuß in die nächste Waschstufe eingespeist.

Das aus der Wäsche mit einer Temperatur von 65 bis 75 °C ablaufende Salpetersäure-/Schwefelsäuregemisch von ca. 30 bis 40 % Gesamtsäure ist mit DNT gesättigt und kann entweder direkt oder nach einer weiteren Aufkonzentrierung auf einen Säuregehalt von bis zu 65 % Gesamtsäure getrennt oder zusammen mit dem bei der Strippung von MNT-Endsäure erhaltenen Gemisch aus dünner Salpetersäure (15 - 30 %) und MNT/DNT in den Nitrierprozeß zurückgeführt werden.

Eine weitere Möglichkeit der Rückführung der Waschsäure aus der Wäsche des Roh-DNT und des bei der Strippung von MNT-Endsäure erhaltenen Gemisches aus dünner Salpetersäure und MNT/DNT besteht darin, beide gemeinsam so aufzukonzentrieren, daß ein Säuregemisch aus Schwefelsäure und Salpetersäure mit einer Konzentration von 50 bis 60 % Gesamtsäure erhalten wird, das gleichfalls zusammen mit dem MNT/DNT aus der Strippung der MNT-Endsäure in die Nitrierung auf der MNT-Stufe zurückgeführt werden kann. Nur noch die Salpetersäure, die aus der DNT-Wäsche in die zweite Waschstufe in die Wäsche mit Alkali gelangt, trägt somit noch zur Abwasserbelastung bei.

Durch diese Maßnahmen wird nicht nur die Belastung des Abwassers aus einer vollständigen Wäsche des Roh-DNT mit Salpetersäure von etwa 1 bis 3 % auf 200 bis 500 ppm bzw. von etwa 20 bis 35 kg Nitrat und Nitrit/ Tonne DNT auf 300 bis 700 g Nitrat und Nitrit/t DNT gesenkt, sondern auch der für eine vollständige Nitrierung in den einzelnen Nitrierstufen notwendige Überschuß an Salpetersäure, der z.T. mit dem Roh-DNT ausgetragen wird und die durch Oxidation der Nitrokresole auf der DNT-Stufe verbrauchte Salpetersäure, die als Nitrose mit der MNT-Endsäure aus der Nitrierung ausgetragen wird, werden so wiedergewonnen und für die Nitrierung nutzbar gemacht.

Die Wäsche wird erfindungsgemäß in mehreren im Gegenstrom geschalteten Waschstufen durchgeführt. Mindestens zwei Waschstufen sind nötig, um ein Ablaufen des Salpetersäure-/Schwefelsäuregemisches von 30 bis 40 % Gesamtsäure als Salpetersäure und eine Rückgewinnung von mindestens 95 % der mit dem Roh-DNT in das Abwasser ausgetragenen $HNO_3$ und Nitrose zu erreichen.

Als Waschapparat kann jede zu einer Gegenstromwäsche geeignete Vorrichtung eingesetzt werden. Bevorzugt wird die Wäsche in Mixer/Settlern durchgeführt werden, wie sie z.B. in der DE-B-11 35 425 beschrieben sind. Die Produktqualität des gewaschenen Roh-DNT wird durch die Rückführung des Waschextraktes aus der sauren Wäsche in die Nitrierung nicht verändert.

**Beispiel**

3400 kg/h rohes DNT wird nach Abtrennung von der DNT-Endsäure in der sauren Wäsche bei einer Temperatur oberhalb des Schmelzpunktes des DNT-Isomerengemisches (60 - 75 °C) in zwei Extraktionsstufen mit verdünnten wäßrigen Lösungen Schwefelsäure, salpetriger Säure ($H_2SO_4$/$HNO_3$/$HNO_2$) gewaschen. Das unbehandelte Roh-DNT mit einem Gehalt von ca. 8,9 kg Schwefelsäure pro Tonne DNT, ca. 17,5 kg Salpetersäure pro Tonne und ca. 9,3 kg Stickstoffdioxid pro Tonne wird in der ersten Extraktionsstufe mit einer Waschsäure der Zusammensetzung 6,25 % Schwefelsäure, 17,06 % Salpetersäure und 0,42 % $HNO_2$ im Volumenverhältnis 1 : 1 DNT : Waschsäure gewaschen. Gleichzeitig wird aus der zweiten Extraktionsstufe soviel Waschsäure mit geringerer Säurekonzentration als in der ersten Extraktionsstufe zugeführt, daß sich die Konzentration der Säuren in der ersten Extraktionsstufe nicht ändert.

In der zweiten Extraktionsstufe wird gleichfalls im Phasenverhältnis 1 : 1 mit einer Waschsäure mit der Konzentration gewaschen, mit der diese Waschsäure aus der zweiten Waschstufe in die erste Waschstufe abgegeben wird. Gleichzeitig wird in die Waschstufe soviel Frischwasser eingespeist, daß sich die Konzentration der Säuren in der Waschsäure durch Extraktion von Säure aus dem DNT nicht ändert.

Die Menge des Frischwassers, das auf der letzten Extraktionsstufe eingespeist wird, wird so gewählt, daß die Konzentrationen der Waschsäure in der ersten Extraktionsstufe im Kontakt mit dem zu waschenden DNT die für diese Waschsäure festgelegte Säurestärke nicht überschritten bzw. die festgelegte Dichte nicht verändert wird.

Um das gewünschte Volumenverhältnis in jeder Waschstufe von 1 : 1 aufrechtzuerhalten, wird die aus dem Scheideteil eines Mixer Settlers nach Trennung der Phasen ablaufende Waschsäure wieder zurück in den Waschteil als Waschsäure eingespeist.

Der Überschuß an Waschsäure in jeder Extraktionsstufe, der durch ständige Zuführung von verdünnter Waschsäure aus der vorhergehenden Extraktionsstufe oder beim letzten Extraktionswäscher durch Einspeisen von Wasser oder Destillat (mit Spuren an $HNO_3$, $HNO_2$ und $H_2SO_4$) aus der Aufkonzentrierung der Waschsäure aus der ersten Waschstufe erhalten wird, wird in die vorhergehende Extraktionsstufe eingespeist.

Die aus der ersten Extraktionsstufe abfließende Waschsäure mit einem Gehalt an 6,25 % $H_2SO_4$, 17,06 % $HNO_3$ und 0,42 % $HNO_2$, die mit DNT/MNT entsprechend der Temperatur der Wäsche gesättigt ist, wird entweder direkt in die Nitrierung gegeben oder in einer Destillationseinrichtung von 23,73 Gew.-% separat oder zusammen mit dem Brüdenkondensat (bestehend aus ca. 20 - 30 % $HNO_3$, ca. 0 - 2 % Schwefelsäure und einem MNT/DNT-Gemisch) aus der Reinigung der MNT-Endsäure auf eine Säurekonzentration von max. 65 % Gesamtsäure, als Salpetersäure bestimmt, aufkonzentriert.

Die aus der Aufkonzentrierung ablaufende Säure von max. 65 % Gesamtsäure wird gleichfalls in die Nitrierung zurückgeführt. Das anfallende Destillat mit ca. 0,3 bis 0,8 % an Säure, vorwiegend Salpetersäure, wird als Waschsäure in die Extraktionswäsche zurückgeführt.

Nach Verlassen der letzten Waschstufe waren die im Roh-DNT enthaltenen stickstoffhaltigen Säuren ($HNO_3$ und $HNO_2$) zu ca. 98 % in die Waschsäuren der Gegenstromwäsche extrahiert und standen damit für die Rückführung in die Nitrierung zur Verfügung.

**Patentansprüche**

**1.** Verfahren zum Entfernen und Wiedergewinnen von Salpetersäure, Schwefelsäure und Stickoxiden aus den bei der Nitrierung von Toluol oder Mononitrotoluolen nach Abtrennung der Nitriersäure anfallenden rohen Dinitrotoluolen,
**dadurch gekennzeichnet,**
**daß** die rohen Dinitrotoluole mit einer verdünnten wäßrigen Lösung von Salpetersäure, Schwefelsäure und salpetriger Säure, deren Dichte geringer ist als die der Dinitrotoluole, mehrstufig im Gegenstrom extrahiert werden, wobei das Volumenverhältnis der Dinitrotoluole zu der wäßrigen Lösung jeweils 1:3 bis 10:1 beträgt und wobei die verdünnte wäßrige Lösung innerhalb jeder Extraktionsstufe im Kreislauf gerührt wird,
wobei das Volumenverhältnis der Dinitrotoluole zu der verdünnten wäßrigen Lösung von Salpetersäure, Schwefelsäure und salpetriger Säure über die Zugabe von Frischwasser in den Extraktionskreislauf der verdünnten Lösung der letzten Extraktionsstufe eingestellt wird und wobei die aus der ersten Extraktionsstufe abgezogene wäßrige Lösung ein Salpetersäure/Schwefelsäure-Gemisch mit 25 bis 40 % Gesamtsäure ist, und
**daß** der wäßrige Extrakt direkt oder nach Aufkonzentrierung in die Nitrierung zurückgeführt wird.

**2.** Verfahren nach Anspruch 1, **dadurch gekennzeichnet, daß** das Volumenverhältnis der rohen Dinitrotoluole zu der wäßrigen Lösung 1:1 bis 4:1 beträgt.

**3.** Verfahren nach Anspruch 1 oder 2, **dadurch gekennzeichnet, daß** zwei- bis vierstufig extrahiert wird.

**4.** Verfahren nach einem der Ansprüche 1 bis 3, **dadurch gekennzeichnet, daß** in allen Extraktionsstufen bei einer Temperatur über der Schmelztemperatur der Dinitrotoluole gearbeitet wird.

**5.** Verfahren nach einem der Ansprüche 1 bis 4, **dadurch gekennzeichnet, daß** das bei der Aufkonzentnerung anfallende Kondensat dem Extraktionskreislauf der verdünnten Lösung der letzten Extraktionsstufe augesetzt wird.

**6.** Verfahren nach einem der Ansprüche 1 bis 5, **dadurch gekennzeichnet, daß** die wäßrige Lösung bis zu einem Gesamtsäuregehalt von 65 %, berechnet als $HNO_3$, konzentriert wird.

**7.** Verfahren nach einem der Ansprüche 1 bis 6, **dadurch gekennzeichnet, daß** die aus der ersten Extraktionsstufe abgezogene wäßrige Lösung zusammen mit der bei der Mononitrotoluol-Endsäurebehandlung anfallenden-Salpetersäure enthaltenden Lösung aufkonzentriert wird.

**Claims**

**1.** A process for removing and recovering nitric acid, sulphuric acid and nitrogen oxides from the raw dinitrotoluenes produced in the nitration of toluene or mononitrotoluenes after separating off the nitrating acid,
**characterised**
**in that** the raw dinitrotoluenes are extracted with a dilute aqueous solution of nitric acid, sulphuric acid and nitrous acid, the density of which is less than that of the dinitrotoluenes, in several stages in a counter-current flow, the volume ratio of the dinitrotoluenes to the aqueous solution in each case being 1 : 3 to 10 : 1 and the dilute aqueous solution being recirculated within each extraction stage, wherein the volume ratio between the dinitrotoluenes and the dilute aqueous solution of nitric acid, sulphuric acid and nitrous acid is adjusted by feeding fresh water into the extraction circuit of the dilute solution of the last extraction step and wherein the aqueous solution withdrawn from the first extraction stage is a nitric acid/sulphuric acid mixture comprising from 25 % to 40 % total acid, and
**in that** the aqueous extract is returned to the nitration stage directly or after being concentrated.

2. A process according to Claim 1, **characterised in that** the volume ratio of the raw dinitrotoluenes to the aqueous solution is 1 : 1 to 4 : 1.

3. A process according to Claim 1 or 2, **characterised in that** extraction is performed in two to four stages.

4. A process according to one of Claims 1 to 3, **characterised in that** in all the extraction stages operation is at a temperature above the melting temperature of the dinitrotoluenes.

5. A process according to one of Claims 1 to 4, **characterised in that** the condensate produced upon the concentration is added to the extraction circulation of the dilute solution of the final extraction stage.

6. A process according to one of Claims 1 to 5, **characterised in that** the aqueous solution is concentrated up to a total acid content of 65 %, calculated as $HNO_3$.

7. A process according to one of Claims 1 to 6, **characterised in that** the aqueous solution withdrawn from the first extraction stage, together with the solution containing nitric acid which is produced in the mononitrotoluene final acid treatment, is concentrated.

## Revendications

1. Procédé d'élimination et de récupération de l'acide nitrique, de l'acide sulfurique et des oxydes d'azote des dinitrotoluènes bruts obtenus par nitration du toluène ou de mononitrotoluènes après séparation du mélange sulfonitrique,
**caractérisé**
**en ce que** les dinitrotoluènes bruts sont extraits à l'aide d'une solution aqueuse diluée d'acide nitrique, d'acide sulfurique et d'acide nitreux dont la densité est inférieure à celle des dinitrotoluènes, en plusieurs étapes à contre-courant, le rapport volumique des dinitrotoluènes par rapport à la solution aqueuse étant chaque fois de 1 : 3 et 10 : 1 et la solution aqueuse diluée étant conduite en cycle à chaque étape d'extraction, où le rapport volumique des dinitrotoluènes par rapport à la solution aqueuse diluée d'acide nitrique, d'acide sulfurique et d'acide nitreux est ajusté par addition d'eau fraîche dans le cycle d'extraction de la solution diluée de la dernière étape d'extraction et où la solution aqueuse récupérée de la première étape d'extraction est un mélange d'acide nitrique et d'acide sulfurique renfermant de 25 % à 40 % d'acide total, et
**en ce que** l'extrait aqueux est reconduit directement ou après reconcentration vers la nitration.

2. Procédé selon la revendication 1, **caractérisé en ce que** le rapport volumique des dinitrotoluènes bruts par rapport à la solution aqueuse est de 1 : 1 à 4 : 1.

3. Procédé selon la revendication 1 ou 2, **caractérisé en ce que** l'extraction est effectuée en deux à quatre étapes.

4. Procédé selon l'une quelconque des revendications 1 à 3, **caractérisé en ce que** dans toutes les étapes d'extraction, on opère à une température supérieure au point de fusion des dinitrotoluènes.

5. Procédé selon l'une quelconque des revendications 1 à 4, **caractérisé en ce que** le produit de condensation résultant de la concentration est ajouté au cycle d'extraction de la solution diluée de la dernière étape d'extraction.

6. Procédé selon l'une quelconque des revendications 1 à 5, **caractérisé en ce que** l'on concentre la solution aqueuse jusqu'à une concentration totale en acide de 65 % exprimé en $HNO_3$.

7. Procédé selon l'une quelconque des revendications 1 à 6, **caractérisé en ce que** la solution aqueuse récupérée de la première étape d'extraction est concentrée en association avec la solution obtenue du traitement à l'acide final de mononitrotoluène contenant de l'acide nitrique.

# EP 0 736 514 B2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4482769 A
- EP 415354 B
- EP 279312 B
- US 4257986 A
- EP 155586 B
- CA 1034603 A
- DE 1135425 B